# EUROPEAN PATENT APPLICATION

(11) **EP 4 726 054 A1**
(43) Date of publication of application: **15.04.2026**
(21) Application number: 24819428.4
(22) Date of filing: 07.06.2024
(51) Int. Cl.: C12Q 1/6813, C07K 16/00, C12M 1/00, C12M 1/34, C12N 15/11, C12N 15/13

(54) **METHOD FOR DETECTING TARGET SUBSTANCE IN SAMPLE, AND KIT THEREFOR**

(30) Priority: 09.06.2023 JP 2023095436
(71) Applicant: Toppan Holdings Inc., Tokyo 110-0016 (JP)
(72) Inventor: KUBO, Yuji, Tokyo 110-0016 (JP)
(74) Representative: Plasseraud IP
(86) International application number: PCT/JP2024/020941
(87) International publication number: WO 2024/253201

(57) **Abstract**

A method for detecting a target substance in a sample includes: a step of incubating a mixed solution containing the sample, a first specific binding substance labeled with a first single-stranded nucleic acid fragment, and a second specific binding substance labeled with a second single-stranded nucleic acid fragment, thereby forming a complex containing the target substance, the first specific binding substance and the second specific binding substance, during which a double-stranded nucleic acid is formed by hybridization of at least part of the first single-stranded nucleic acid fragment with at least part of the second single-stranded nucleic acid fragment; and a step of detecting formation of the double-stranded nucleic acid, wherein detection of the double-stranded nucleic acid being formed indicates that the target substance is present in the sample, and the mixed solution further contains 2-methacryloyloxyethyl phosphorylcholine copolymer, or the mixed solution further contains casein and a nonionic surfactant.

## Description

### [Technical Field]

The present invention relates to a method for detecting a target substance in a sample and a kit therefor.

The present application is based on and claims the benefit of priority from Japanese Patent Application No. 2023-095436 filed June 9, 2023, the content of which is incorporated herein by reference.

### [Background Art]

With the advent of comprehensive genetic analysis technologies, represented by next-generation sequencers, many genetic alterations and mechanisms of disease onset are becoming clearer, but at the same time, numerous variants of unknown significance (hereinafter, referred to as "VUS") have been identified.

In current genomic medicine, the low diagnostic rate and drug delivery efficiency through genetic analysis are problematic. One of the reasons for this is the large number of VUS cases reported, and there is an urgent need to determine the significance of VUS and evaluate the VUS.

To determine the significance of VUS and evaluate the VUS, various scores, including Combined Annotation Dependent Depletion (https://cadd.gs.washington.edu/) score and MutPred2 (https://mutpred.mutdb.org/) score, are calculated and can be referenced. However, computational predictions cannot be used as the sole evidence for evaluating pathogenicity because they may not match the actual phenotype.

One established method of experimentally evaluating VUS involves creating a plasmid with a mutation in the target gene, introducing the plasmid into cells to synthesize a protein, phosphorylating the protein synthesized in the cells, and then detecting the cell extract containing the phosphorylated protein using Western blotting or the like to qualitatively evaluate the VUS. However, such an evaluation method requires a lot of time, effort and cost.

Conventionally, protein quantitative detection has been performed by enzyme-linked immunosorbent assay (ELISA) or the like, and nucleic acid quantification has been performed by the real-time PCR method or the like.

As a method of detecting a target substance with higher accuracy, techniques for causing enzymatic reactions in a large number of microcompartments are being considered. These techniques are called digital quantification. Digital quantification includes digital ELISA and digital PCR.

In digital quantification, a sample solution is divided into an extremely large number of micro-solutions. Then, signals from the individual micro-solutions are binarized and only whether the target substance is present is determined to measure the number of molecules of the target substance. The digital quantification can significantly improve the detection sensitivity and the quantitativeness compared to conventional methods such as ELISA, real-time PCR, and the like.

For example, NPL 1 describes a microwell array with microwells and channels for supplying reagents, etc., and describes performing a digital ELISA using the microwell array.

PTL 1 describes a proximity ligation assay (PLA) which is a method of detecting a protein using an antibody modified with an oligonucleotide. This method utilizes a PCR method or RCA method for the detection.

PTL 2 describes a method of detecting protein interactions between two molecules using an antibody modified with an oligonucleotide. This method also utilizes a PCR method or RCA method for the detection.

NPL 3 describes a microwell array with microwells and channels for supplying reagents, etc., and describes expressing signal transduction using cells in the microwell array to detect phosphorylated proteins. The state of signal transduction is monitored by detecting phosphorylated proteins.

Alpha SureFire (PerkinElmer: https://www.perkinelmer.co.jp/assays/tabid/346/Default.aspx) is an assay system with which kinase activity can be detected on a cell-by-cell basis without the need of washing procedures. In this system, phosphorylated proteins are detected from cell extracts.

In order to detect target substances with high sensitivity by ELISA or the like, reaction solutions that effectively suppress non-specific adsorption to various substances are being considered. These reaction solutions are called blocking agents.

As the blocking agents, those containing, as main components, biologically derived proteins such as bovine serum albumin, casein and gelatin, highly hydrophilic inorganic microparticles, phosphorylcholine group-containing polymer, etc. have been conventionally used.

In NPL 4, 2-methacryloyloxyethyl phosphorylcholine copolymer (MPC polymer) is used as a blocking agent to prevent non-specific adsorption.

### [Citation List]

### [Patent Literature]

PTL 1: JP 2020-122799 A
PTL 2: US 10,465,235 B

### [Non-Patent Literature]

NPL 1: Kan C. W., et al., Isolation and detection of single molecules on paramagnetic beads using sequential fluid flows in microfabricated polymer array assemblies, Lab on a Chip, 12 (5), 977-985, 2012.
NPL 2: Mohammed H., et al., Approaches for Assessing and Discovering Protein Interactions in Cancer., Mol Cancer Res, 11 (11), 1295-1302, 2013.
NPL 3: Blazek M., et al., Proximity Ligation Assay for High-content Profiling of Cell Signaling Pathways on a Microfluidic Chip., Molecular & Cellular Proteomics 12: 10.1074/mcp.M113.032821, 3898-3907, 2013.
NPL 4: Y. Iwasaki, K. Ishihara., Phosphorylcholine-containing polymers for biomedical applications., Anal Bioanal Chem 381, 534-546, 2005.

### [Summary of Invention]

### [Technical Problem]

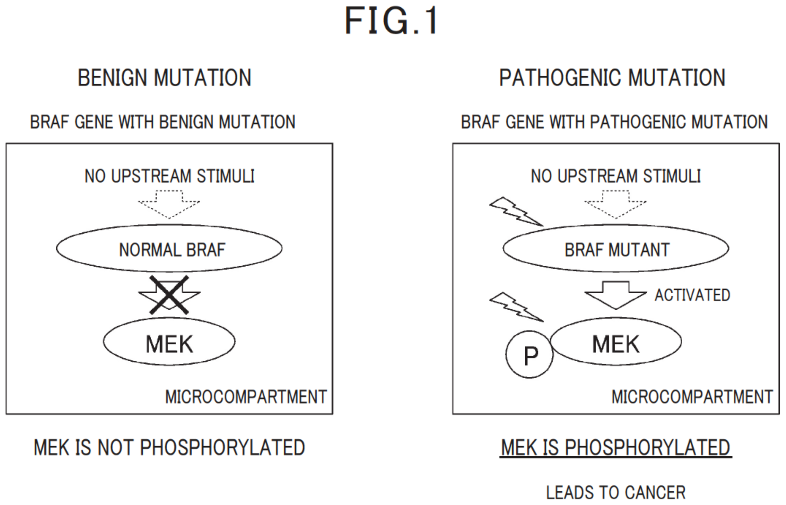

Living organisms have mechanisms for transmitting external stimuli into cells. In these mechanisms, various proteins temporarily bind to each other and transmit information. This information transmission system, which is referred to as a signal transduction system, is assumed by various protein molecules that mediate stimuli. Fig. 1 shows, as an example, part of signal transduction of mitogen-activated protein kinase (MAPK).

Fig. 1 shows, as an example, the case where the mutant protein is BRAF, and the variants of unknown significance (VUS) in the BRAF gene are either benign mutations or pathogenic mutations. When the VUS is a benign mutation, MEK, the substrate of BRAF, is not phosphorylated unless there are upstream stimuli, as shown in Fig. 1 left. In contrast, when the VUS is a pathogenic mutation, BRAF is constantly activated and phosphorylates its substrate, MEK, even in the absence of upstream stimuli, as shown in Fig. 1 right. Such BRAF causes cancer.

The present invention has been made to provide a method for detecting a target substance in a sample and a kit therefor. According to the present invention, for example, a functional analysis (evaluation) of VUS can be performed easily, efficiently, and in a short period of time.

### [Solution to Problem]

The present invention includes the following aspects.
[1] A method for detecting a target substance in a sample, the method including: a step of incubating a mixed solution containing the sample, a first specific binding substance for the target substance, the first specific binding substance being labeled with a first single-stranded nucleic acid fragment, and a second specific binding substance for the target substance, the second specific binding substance being labeled with a second single-stranded nucleic acid fragment, thereby forming a complex containing the target substance, the first specific binding substance and the second specific binding substance when the target substance is present in the sample, during which a double-stranded nucleic acid is formed by hybridization of at least part of the first single-stranded nucleic acid fragment with at least part of the second single-stranded nucleic acid fragment; and a step of detecting formation of the double-stranded nucleic acid, wherein
   detection of the double-stranded nucleic acid being formed indicates that the target substance is present in the sample, and the mixed solution further contains 2-methacryloyloxyethyl phosphorylcholine copolymer (MPC polymer), or the mixed solution further contains casein and a nonionic surfactant.
[2] The method according to [1], wherein the MPC polymer is a copolymer of 2-methacryloyloxyethyl phosphorylcholine and a comonomer having at least one group selected from the group consisting of a hydrophobic group, a hydrophilic group, an anionic group and a cationic group.
[3] The method according to [1] or [2], wherein the MPC polymer in the mixed solution has a concentration of 0.01 to 0.5% (w/v).
[4] The method according to any of [1] to [3], wherein the incubation of the mixed solution is performed in a well, and the well has a volume of 10 fL to 100 pL.
[5] The method according to any of [1] to [4], wherein the first single-stranded nucleic acid fragment and the second single-stranded nucleic acid fragment each have a length of 10 to 200 bases.
[6] The method according to any of [1] to [5], wherein the step of detecting formation of the double-stranded nucleic acid is performed through an invasive cleavage assay.
[7] A kit for detecting a target substance in a sample, the kit including: a well array having a plurality of wells; a first specific binding substance for the target substance, the first specific binding substance being labeled with a first single-stranded nucleic acid fragment; a second specific binding substance for the target substance, the second specific binding substance being labeled with a second single-stranded nucleic acid fragment; and a buffer containing an MPC polymer or containing casein and a nonionic surfactant.
[8] The kit according to [7], further including a sealing liquid for sealing openings of the wells.
[9] The kit according to [7] or [8], further including a reagent for detecting a double-stranded nucleic acid formed by hybridization of at least part of the first single-stranded nucleic acid fragment with at least part of the second single-stranded nucleic acid fragment.

The present invention includes the following aspects.
[11] A method for detecting a target substance in a sample, the method including:
   a step of incubating a mixed solution containing the sample, a first specific binding substance for the target substance, the first specific binding substance being labeled with a first single-stranded nucleic acid fragment, a second specific binding substance for the target substance, the second specific binding substance being labeled with a second single-stranded nucleic acid fragment, and 2-methacryloyloxyethyl phosphorylcholine copolymer, thereby forming a complex containing the target substance, the first specific binding substance and the second specific binding substance, during which a double-stranded nucleic acid is formed by hybridization of at least part of the first single-stranded nucleic acid fragment with at least part of the second single-stranded nucleic acid fragment; and
   a step of detecting formation of the double-stranded nucleic acid, wherein
   detection of the double-stranded nucleic acid being formed indicates that the target substance is present in the sample.
[12] The method according to [11], wherein the 2-methacryloyloxyethyl phosphorylcholine copolymer is a copolymer of 2-methacryloyloxyethyl phosphorylcholine and a comonomer having at least one group selected from the group consisting of a hydrophobic group, a hydrophilic group, an anionic group and a cationic group.
[13] The method according to [11] or [12], wherein the MPC polymer in the mixed solution has a concentration of 0.01 to 0.5% (w/v).
[14] A method for detecting a target substance in a sample, the method including:
   a step of incubating a mixed solution containing the sample, a first specific binding substance for the target substance, the first specific binding substance being labeled with a first single-stranded nucleic acid fragment, a second specific binding substance for the target substance, the second specific binding substance being labeled with a second single-stranded nucleic acid fragment, and a buffer containing casein and a nonionic surfactant, thereby forming a complex containing the target substance, the first specific binding substance and the second specific binding substance, during which a double-stranded nucleic acid is formed by hybridization of at least part of the first single-stranded nucleic acid fragment with at least part of the second single-stranded nucleic acid fragment; and
   a step of detecting formation of the double-stranded nucleic acid, wherein
   detection of the double-stranded nucleic acid being formed indicates that the target substance is present in the sample.
[15] The method according to any of [11] to [14], wherein the incubation of the mixed solution is performed in a well, and the well has a volume of 10 fL to 100 pL.
[16] The method according to any of [11] to [15], wherein the first single-stranded nucleic acid fragment and the second single-stranded nucleic acid fragment each have a length of 10 to 200 bases.
[17] The method according to any of [11] to [16], wherein the step of detecting formation of the double-stranded nucleic acid is performed through an invasive cleavage assay.
[18] A kit for detecting a target substance in a sample, the kit including: a well array having a plurality of wells; a first specific binding substance for the target substance, the first specific binding substance being labeled with a first single-stranded nucleic acid fragment; a second specific binding substance for the target substance, the second specific binding substance being labeled with a second single-stranded nucleic acid fragment; and a 2-methacryloyloxyethyl phosphorylcholine copolymer.
[19] A kit for detecting a target substance in a sample, the kit including: a well array having a plurality of wells; a first specific binding substance for the target substance, the first specific binding substance being labeled with a first single-stranded nucleic acid fragment; a second specific binding substance for the target substance, the second specific binding substance being labeled with a second single-stranded nucleic acid fragment; and a buffer containing casein and a nonionic surfactant.
[20] The kit according to [18] or [19], further including a sealing liquid for sealing openings of the wells.
[21] The kit according to any of [18] to [20], further including a reagent for detecting a double-stranded nucleic acid formed by hybridization of at least part of the first single-stranded nucleic acid fragment with at least part of the second single-stranded nucleic acid fragment.

### [Advantageous Effects of Invention]

According to the present invention, a method for detecting a target substance in a sample and a kit therefor can be provided. According to the present invention, for example, a functional analysis (evaluation) of VUS can be performed easily, efficiently, and in a short period of time.

### [Brief Description of Drawings]

Fig. 1 is a set of diagrams illustrating a benign mutation and a pathogenic mutation.
Fig. 2 is a schematic diagram illustrating a method for detecting a target substance.
Fig. 3 is a schematic diagram illustrating a method for detecting a target substance.
Fig. 4 is a schematic cross-sectional view illustrating an example of a fluidic device.
Fig. 5 is a schematic cross-sectional view illustrating a method for detecting a target substance.
Fig. 6 is a schematic cross-sectional view illustrating a method for detecting a target substance.
Fig. 7 is a schematic cross-sectional view illustrating an example of a fluidic device.
Fig. 8 is a schematic cross-sectional view illustrating a method for detecting a target substance.
Fig. 9 is a schematic cross-sectional view illustrating a method for detecting a target substance.
Fig. 10 is a schematic diagram illustrating an example of an Invasive Cleavage Assay (ICA) method.
Fig. 11 is a schematic diagram illustrating a method of evaluating the activity of a protein of interest.
Fig. 12 is a graph showing the results of Experimental Example 1.
Fig. 13 is a graph showing the results of Experimental Example 1.
Fig. 14 is a graph showing the results of Experimental Example 2.
Fig. 15 is a graph showing the results of Experimental Example 2.
Fig. 16 is a graph showing the results of Experimental Example 3.
Fig. 17 is a graph showing the results of Experimental Example 3.
Fig. 18 is a graph showing the results of Experimental Example 3.
Fig. 19 is a graph showing the results of Experimental Example 3.
Fig. 20 is a graph showing the results of Experimental Example 3.
Fig. 21 is a graph showing the results of Experimental Example 4.

### [Description of Embodiments]

With reference to the drawings as appropriate, embodiments of the present invention will be described in detail. Throughout the drawings, the same or corresponding parts are denoted by the same or corresponding reference signs, and repeated description is omitted. The dimension ratios in some drawings are exaggerated for convenience of illustration, and are not necessarily to scale.

### [Method for Detecting Target Substance in Sample]

In one embodiment, the present invention provides a method for detecting a target substance in a sample, the method including: a step (a) of incubating a mixed solution containing the sample, a first specific binding substance for the target substance, the first specific binding substance being labeled with a first single-stranded nucleic acid fragment, and a second specific binding substance for the target substance, the second specific binding substance being labeled with a second single-stranded nucleic acid fragment, thereby forming a complex containing the target substance, the first specific binding substance and the second specific binding substance when the target substance is present in the sample, during which a double-stranded nucleic acid is formed by hybridization of at least part of the first single-stranded nucleic acid fragment with at least part of the second single-stranded nucleic acid fragment; and a step (b) of detecting formation of the double-stranded nucleic acid, wherein detection of the double-stranded nucleic acid being formed indicates that the target substance is present in the sample, and the mixed solution further contains 2-methacryloyloxyethyl phosphorylcholine copolymer (hereinafter, also referred to as MPC polymer), or the mixed solution further contains casein and a nonionic surfactant.

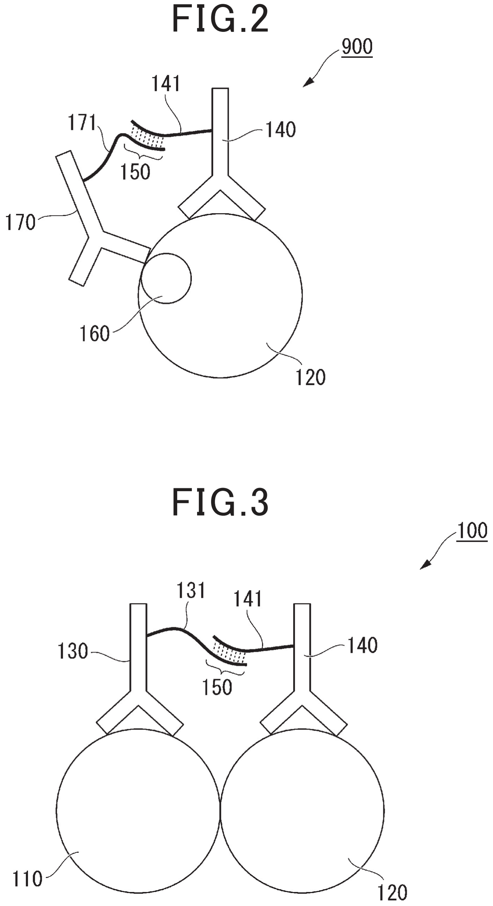

Fig. 2 is a schematic diagram illustrating a method according to the present embodiment. Fig. 2 shows an example in which a phosphorylated protein 120 is detected as a target substance. In Fig. 2, a phosphate group is indicated by reference sign 160.

As shown in Fig. 2, in the method of the present embodiment, in step (a) first, a mixed solution containing a sample, a first specific binding substance 140 for a target substance 120, the first specific binding substance 140 being labeled with a first single-stranded nucleic acid fragment 141, and a second specific binding substance 170 for the target substance 120, the second specific binding substance 170 being labeled with a second single-stranded nucleic acid fragment 171, is incubated. The first specific binding substance 140 and the second specific binding substance 170 recognize different epitopes on the target substance 120. In the example of Fig. 2, the specific binding substance 170 recognizes a region containing the phosphate group 160 of the phosphorylated protein 120.

As a result, when the target substance 120 is present in the sample, a complex 900 is formed containing the target substance 120, the first specific binding substance 140 and the second specific binding substance 170, during which a double-stranded nucleic acid (also referred to as double-stranded nucleic acid region) 150 is formed by hybridization of at least part of the first single-stranded nucleic acid fragment 141 with at least part of the second single-stranded nucleic acid fragment 171.

Subsequently, in step (b), formation of the double-stranded nucleic acid 150 is detected. Detection of the double-stranded nucleic acid 150 being formed indicates that the target substance 120 is present in the sample. Detection of the formation of the double-stranded nucleic acid 150 will be described later.

Fig. 3 is a schematic diagram illustrating another embodiment according to the present embodiment. In the example of Fig. 3, the target substance is a conjugate of a protein 110 and a protein 120. That is, in the example of Fig. 3, it is possible to evaluate whether the protein 110 and the protein 120 bind to each other (i.e., interact with each other).

In the example of Fig. 3, in step (a) first, a mixed solution containing a sample, a first specific binding substance 140 for a protein 120, the first specific binding substance 140 being labeled with a first single-stranded nucleic acid fragment 141, and a second specific binding substance 130 for a protein 110, the second specific binding substance 130 being labeled with a second single-stranded nucleic acid fragment 131, is incubated.

As a result, when a conjugate of the protein 110 and the protein 120 is present in the sample, a complex 100 is formed containing the conjugate of the protein 110 and the protein 120 as the target substance, the first specific binding substance 140 and the second specific binding substance 130, during which a double-stranded nucleic acid (also referred to as double-stranded nucleic acid region) 150 is formed by hybridization of at least part of the first single-stranded nucleic acid fragment 141 with at least part of the second single-stranded nucleic acid fragment 131.

Subsequently, in step (b), formation of the double-stranded nucleic acid 150 is detected. Detection of the double-stranded nucleic acid 150 being formed indicates that the conjugate of the protein 110 and the protein 120 as the target substance is present in the sample. That is, detection of the double-stranded nucleic acid 150 being formed indicates that the protein 110 and the protein 120 interact with each other.

The method of Fig. 3 may be applied to the case where the protein of interest 110 does not bind to the target protein 120 (i.e., they do not interact with each other), and in that case, formation of the double-stranded nucleic acid 150 is not detected. Detection of the formation of the double-stranded nucleic acid 150 will be described later.

As will be described later in the examples, when the mixed solution further contains an MPC polymer, it can reduce the time required for detecting the target substance.

Further, as will be described later in the examples, when the mixed solution further contains casein and a nonionic surfactant, it can increase the signal-to-noise ratio (also referred to as S/N).

Further, as will be described later in the examples, when the mixed solution further contains casein, a nonionic surfactant and tris(hydroxymethyl)aminomethane, it can not only increase the signal-to-noise ratio (S/N), but also shorten the time required for detecting the target substance.

According to the method of the present embodiment, mutant protein activity can be evaluated easily and in a short period of time. For example, a functional analysis (i.e., evaluation) of VUS can be performed easily, efficiently, and in a short period of time.

MPC polymer is a polymer of 2-methacryloyloxyethyl phosphorylcholine (MPC) having a phospholipid polar group (also referred to as phosphorylcholine group) and a methacryloyl group in a molecule. MPC polymers mimic a biological membrane and have high biocompatibility, such as extremely low interaction with biological components, such as proteins and blood cells, and excellent antithrombogenic properties. MPC polymers can be copolymerized with various comonomers and impart various properties depending on the type of comonomer.

The MPC polymer may be a copolymer with a comonomer having at least one group selected from the group consisting of a hydrophobic group, a hydrophilic group, an anionic group and a cationic group. The MPC polymer that can be used may be a commercially available product, and specific examples thereof include Lipidure (registered trademark)-BL series (NOF Corporation).

The MPC polymer that can be suitably used may be one having a surface tension of 70 to 80×10⁻³ N/m as measured at 25°C using a 0.1 wt% aqueous solution. Alternatively, the MPC polymer that can be suitably used may be one having a kinematic viscosity of 3 cSt or less, or 20 to 30 cSt as measured at 25°C using a 1 wt% aqueous solution.

The surface tension of a 0.1 wt% aqueous solution of the MPC polymer can be measured using a surface tensiometer (for example, DY-700, manufactured by Kyowa Interface Science Co., Ltd.).

The kinematic viscosity of a 0.1 wt% aqueous solution of the MPC polymer can be measured using a viscometer (for example, Cannon Fenske Reverse Flow SF No. 150, manufactured by Sibata Scientific Technology Ltd.).

In the method of the present embodiment, the concentration of the MPC polymer in the mixed solution is preferably 0.01 to 0.5% (w/v), more preferably 0.01 to 0.1% (w/v), and even more preferably 0.01 to 0.05% (w/v).

Casein is a type of phosphoprotein naturally found in milk and cheese. The concentration of casein in the mixed solution is preferably about 0.0025 to 1 mass%, and more preferably about 0.01 to 1 mass%.

Nonionic surfactant is a surfactant which does not contain an ionic group as a polar group, and examples thereof include acetylene glycol-based surfactants such as ethylene oxide and/or propylene oxide adducts of acetylene glycol (specifically, ethylene oxide and/or propylene oxide adducts such as 2,4,7,9-tetramethyl-5-decyne-4,7-diol, and 3,6-dimethyl-4-octyne-3,6-diol); acetylene alcohol-based surfactants such as ethylene oxide and/or propylene oxide adducts of acetylene alcohol (specifically, ethylene oxide and/or propylene oxide adducts such as 3,5-dimethyl-1-hexyn-3-ol); ether-based surfactants such as polyoxyethylene nonylphenyl ether, polyoxyethylene octylphenyl ether, polyoxyethylene dodecylphenyl ether, polyoxyethylene alkyl allyl ether, polyoxyethylene oleyl ether, polyoxyethylene lauryl ether, polyoxyethylene alkyl ether and polyoxyalkylene alkyl ether; ester-based surfactants such as polyoxyethylene oleic acid, polyoxyethylene oleic acid ester, polyoxyethylene distearic acid ester, sorbitan laurate, sorbitan monostearate, sorbitan monooleate, sorbitan sesquioleate, polyoxyethylene monooleate and polyoxyethylene stearate; polyether-modified siloxane-based surfactants such as dimethylpolysiloxane; and other fluorine-containing surfactants such as fluorine alkyl ester and perfluoroalkyl carboxylate. The concentration of the nonionic surfactant in the mixed solution is preferably about 0.0025 to 1 mass%, and more preferably about 0.01 to 1 mass%.

Tris(hydroxymethyl)aminomethane is a type of buffer. Tris(hydroxymethyl)aminomethane may be a salt. Examples of the salt of tris(hydroxymethyl)aminomethane include tris(hydroxymethyl)aminomethane hydrochloride. The concentration of tris(hydroxymethyl)aminomethane in the mixed solution is preferably about 0.0025 to 1 mass%, and more preferably about 0.01 to 1 mass%.

From the perspective that at least part of the first single-stranded nucleic acid fragment 141 is required to be able to hybridize with at least part of the second single-stranded nucleic acid fragment 171 (131), the first single-stranded nucleic acid fragment 141 may have a length of 10 to 200 bases. Similarly, the second single-stranded nucleic acid fragment 171 (131) may also have a length of 10 to 200 bases. The length of the double-stranded nucleic acid 150 formed by hybridization of at least part of the first single-stranded nucleic acid fragment 141 with at least part of the second single-stranded nucleic acid fragment 171 (131) is preferably about 7 to 30 bases, and may be, for example, 9, 12 or 15 bases.

In the example of Fig. 3, the protein 110 may be a protein having variants of unknown significance (VUS). The protein 110 may be, for example, a kinase. More specific examples of the target protein include proteins in intracellular signal transduction pathways, such as BRAF, A-RAF, Raf, MAP3K 4/12, MAP3K11, ASK1 and TAK1. In this case, whether a kinase having VUS is in a constantly activated state can be evaluated based on whether it binds to the protein 120.

Alternatively, the detection shown in the example of Fig. 2 can be performed in the presence of a kinase having VUS (protein 110) so that whether the kinase having VUS (protein 110) is in a constantly activated state can be evaluated by detecting phosphorylation of the protein 120.

The method of the present embodiment can be performed using digital quantification. In this case, the incubation of the mixed solution is preferably performed in wells, and the wells preferably constitute a well array in which a plurality of wells are arranged. The well array is preferred to be disposed in a channel of a fluidic device.

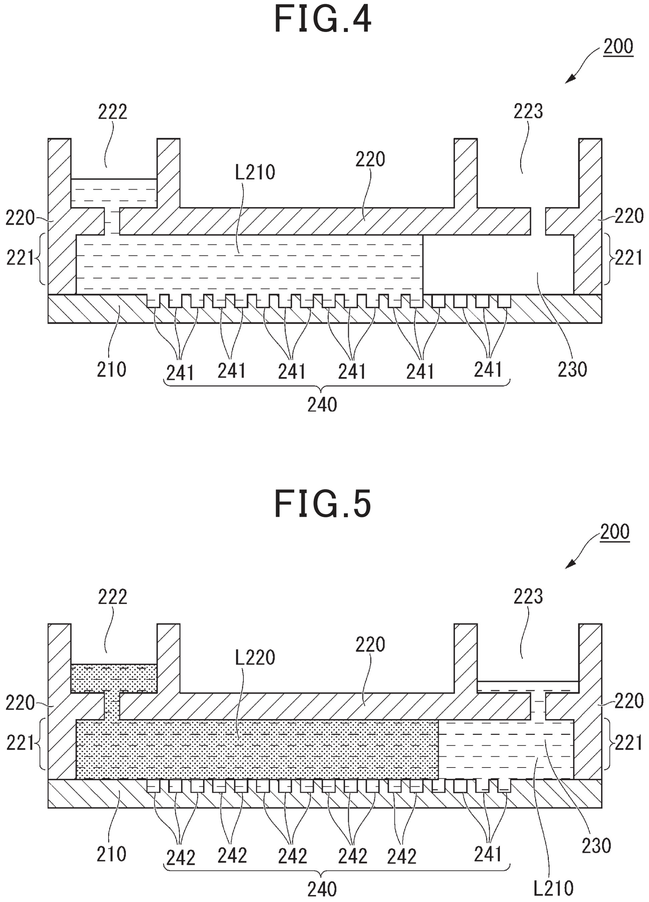

Fig. 4 is a schematic cross-sectional view illustrating an example of a fluidic device with which the method of the present embodiment can be preferably performed. As shown in Fig. 4, a fluidic device 200 includes a substrate 210 and a lid member 220 disposed facing the substrate 210. The lid member 220 has a projection 221, and an end of the projection 221 is in contact with the substrate 210. In the fluidic device 200, a well array 240 is integrally formed on one surface of the substrate 210, and faces the lid member 220. The well array 240 includes a plurality of wells 241. The lid member 220 may be welded or adhered to the substrate 210.

The wells 241 are open to a surface of the substrate 210. The shape, size and arrangement of the wells 241 are not particularly limited, but the wells 241 are preferred to be microwells with small volumes. For example, the volume of each well 241 may be approximately 10 fL to 100 pL, and preferably 50 fL to 1,200 fL. In the fluidic device 200, the plurality of wells 241 with the same shape and size constitute the well array 240. The same shape and size may refer to the same shape and volume required for the digital quantification, accepting a manufacturing error variation.

Each well 241 may have a diameter of approximately 1 µm to 10 µm and a depth of approximately 1 µm to 10 µm, for example. The arrangement of the wells 241 is not particularly limited, and may be arranged, for example, in a triangular lattice shape or a square lattice shape, or may be randomly arranged.

In the fluidic device 200, the presence of the projection 221 forms a space between the well array 240 and the lid member 220. The space provides a channel 230. The channel 230 functions as a path for supplying a dispersion of a protein as a target substance, a first specific binding substance, a second specific binding substance, etc., and for supplying a sealing liquid described later. The shape, structure, volume, and the like of the channel 230 are not particularly limited, but the height of the channel 230 (the distance between the surface of the substrate 210 and the surface of the lid member 220 facing the substrate 210) may be, for example, 500 µm of less, preferably 300 µm or less, more preferably 200 µm or less, and even more preferably 100 µm or less.

The projection 221 may be formed integrally with the lid member 220. For example, the lid member 220 can be formed by molding a thermoplastic resin fluid into a plate shape with a projection 221 using a mold. The lid member 220 may be provided with an inlet port 222 and an outlet port 223 for reagents.

When the lid member 220 has the projection 221, the lid member 220 and the substrate 210 are overlapped with each other so that the projection 221 is brought into contact with the surface of the substrate 210 to which the wells 241 are open. As a consequence, the space between the lid member 220 and the substrate 210 serves as a channel 230. The lid member 220 and the substrate 210 may be welded together by laser welding or the like.

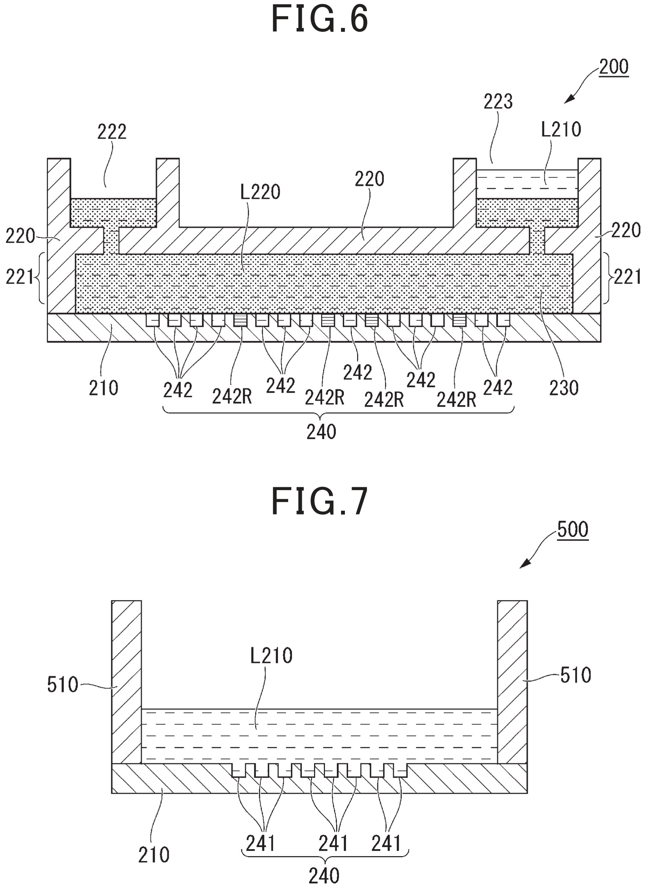

The fluidic device used for the method of the present embodiment is not limited to the fluidic device 200 described above. Fig. 7 is a schematic cross-sectional view illustrating an example of the fluidic device. As shown in Fig. 7, a fluidic device 500 includes a substrate 210 and a wall member 510. In the fluidic device 500, a well array 240 is integrally formed on one surface of the substrate 210. The well array 240 includes a plurality of wells 241. The fluidic device 500 differs from the fluidic device 200 mainly in that it does not include a lid member 220.

In the fluidic device 200, the lid member 220 is formed integrally with the projection 221. However, the lid member 220 and the projection 221 may be molded as separate members.

Further, in the fluidic device 200 and the fluidic device 500 described above, the well array 240 is integrally formed on one surface of the substrate 210. However, the well array may not necessarily be formed integrally with the substrate 210. For example, a well array 240 formed separately from the fluidic device may be disposed on the substrate 210 of the fluidic device. Alternatively, a resin layer may be laminated on the surface of the substrate 210, and a well array may be formed in the resin layer by etching or the like.

In the fluid device, the substrate 210 may be formed using, for example, a resin. The type of the resin is not particularly limited, but it is preferred to use a resin that is resistant to reagents and sealing liquids. Further, when a signal to be detected is fluorescence, it is preferred to use a resin having low autofluorescence. Examples of the resin include, but are not limited to, cycloolefin polymers, cycloolefin copolymers, silicones, polypropylenes, polycarbonates, polystyrenes, polyethylenes, polyvinyl acetates, fluororesins, amorphous fluororesins, and the like.

The substrate 210 may be provided with a plurality of wells 241 formed on one surface thereof in the thickness direction. The wells may be formed using a resin by injection molding, thermal imprinting, optical imprinting, or the like.

Alternatively, for example, a fluororesin may be laminated on the substrate 210, and the fluororesin may be processed by etching or the like to form a well array. The fluororesin that can be used may be, for example, CYTOP (registered trademark) (Asahi Glass Co., Ltd.) or the like.

When the fluidic device includes a lid member 220, the material of the lid member 220 is preferably a resin with low autofluorescence, and examples thereof include thermoplastic resins such as cycloolefin polymers and cycloolefin copolymers.

The lid member 220 may be formed of a material that does not transmit light with a wavelength near the wavelength detected in fluorescence observation for signals, or a material that is completely opaque to light. For example, the lid member 220 may be made of a thermoplastic resin with an addition of carbon, metal particles, etc.

Next, referring to Figs. 4 to 6, a method of the present embodiment will be described, taking an example of using the fluidic device 200. Here, a case where the target substance 120 is detected as in the example of Fig. 2 will be described.

A method of the present embodiment is a method for detecting a target substance 120 in a sample, the method including: a step (a) of incubating a mixed solution containing the sample, a first specific binding substance 140 for the target substance 120, the first specific binding substance 140 being labeled with a first single-stranded nucleic acid fragment 141, and a second specific binding substance 170 for the target substance 120, the second specific binding substance 170 being labeled with a second single-stranded nucleic acid fragment 171, thereby forming a complex 900 containing the target substance 120, the first specific binding substance 140 and the second specific binding substance 170 when the target substance 120 is present in the sample, during which a double-stranded nucleic acid 150 is formed by hybridization of at least part of the first single-stranded nucleic acid fragment 141 with at least part of the second single-stranded nucleic acid fragment 171; and a step (b) of detecting formation of the double-stranded nucleic acid 150, wherein detection of the double-stranded nucleic acid 150 being formed indicates that the target substance 120 is present in the sample.

The method of the present embodiment is the same as that in the case where the target substance is a conjugate of a protein 110 and a protein 120, as in the example of Fig. 3, except that a second specific binding substance 130 for the protein 110 is used as the second specific binding substance.

The mixed solution further contains an MPC polymer, or contains casein and a nonionic surfactant. Preferably, the mixed solution further contains casein, a nonionic surfactant and tris(hydroxymethyl)aminomethane.

As will be described later in the examples, according to the method of the present embodiment, it is possible to reduce the time required for detecting the target substance in the sample, or increase the signal-to-noise ratio (S/N). According to the present invention, for example, a functional analysis (evaluation) of VUS can be performed easily, efficiently, and in a short period of time.

An example in which the mixed solution contains an MPC polymer will be described below. First, as shown in Fig. 4, a mixed solution L210 is introduced from the inlet port 222 of the fluidic device 200 and supplied into the channel 230. The mixed solution L210 contains an MPC polymer, and is a dispersion of a target substance 120, a first specific binding substance 140 and a second specific binding substance 170, and also contains a reagent for detecting formation of a double-stranded nucleic acid 150.

The mixed solution L210 supplied into the channel 230 comes into contact with the well array 240. Then, the mixed solution L210 is accommodated in the wells 241. As a result, the MPC polymer, the target substance 120, the first specific binding substance 140, the second specific binding substance 170 and a reagent for detecting formation of a double-stranded nucleic acid 150 are introduced into the wells 241. If the sample contains the target substance 120, the mixed solution L210 contains a complex 900 containing the target substance 120, the first specific binding substance 140 and the second specific binding substance 170.

The number of units of the complex 900 introduced into each well 241 is not particularly limited, but it is preferred that 1 or less, i.e., 0 or 1 unit of the complex 900, is introduced into each well 241. Thus, units of the complex 900 can be detected on an individual basis, i.e., digital quantification can be performed. The complex 900 does not necessarily need to be introduced into all wells of the well array.

The means of introducing the complex 900 into the wells is not particularly limited, and examples thereof include a method of settling the complex 900 in the fluidic device (specifically, in the channel) by its own weight and distributing it into the wells. Alternatively, a substance (hereinafter, also referred to as a capture substance) that captures the complex 900 may be used, and the capture substance may be bound to the complex 900, which is less likely to settle by its own weight, during the supply of the complex 900, or the capture substance can be immobilized in the wells in advance to capture the supplied complex 900, thereby improving the efficiency of introducing the complex 900 into the wells.

The step of binding the capture substance to the complex 900 can be performed at any time during the method of the present embodiment. For example, this step may be performed by bringing the complex 900 into contact with the capture substance in a sample tube before the complex 900 is introduced into the wells 241. Alternatively, the complex 900 may be introduced into the wells after the capture substance is introduced into the wells 241 so that the capture substance is brought into contact with the complex 900 in the wells.

The capture substance is a substance capable of capturing the complex 900. The capture substance may be, for example, a conjugate of a solid phase and a specific binding substance for the complex 900.

The solid phase may be particles, membranes, substrates, or the like. One type, or two or more types of the specific binding substance may be used for the complex 900. For example, three, four, or five or more specific binding substances may be used.

The particles are not particularly limited, but may be polymer particles, magnetic particles, glass particles, or the like. The particles are preferred to undergo surface treatment to avoid nonspecific adsorption. In order to immobilize the specific binding substance, it is preferred to use particles having a functional group such as a carboxyl group on the surfaces. More specifically, Magnosphere LC300 (product name) manufactured by JSR Corporation, or the like can be used.

Examples of the first specific binding substance 140, the second specific binding substance 170, and the specific binding substance in the capture substance include antibodies, antibody fragments, and aptamers. Examples of the antibody fragments include Fab, F(ab')₂, Fab', single chain antibodies (scFvs), disulfide stabilized antibodies (dsFvs), dimeric V region fragments (diabodies), and peptides including CDRs. The antibodies may be monoclonal or polyclonal. Alternatively, the antibodies may be commercially available antibodies.

The method of labelling a specific binding substance with a single-stranded nucleic acid fragment may include methods using cross-linking agents. The specific binding substance may be labelled with a single-stranded nucleic acid fragment via a linker molecule. Examples of the linker include polyethylene chains, hydrocarbon chains and peptides, but are not specifically limited thereto. The single-stranded nucleic acid fragment may be DNA or RNA. The single-stranded nucleic acid fragment may contain an artificial nucleic acid such as BNA or LNA.

The method of immobilizing the specific binding substance on particle surfaces is not particularly limited, and may be a method using physical adsorption, a method using chemical bonding, a method using avidin-biotin binding, a method using a bond of protein G or protein A to an antibody, or the like. The method using physical adsorption may be a method of immobilizing the specific binding substance on particle surfaces using hydrophobic interaction or electrostatic interaction. The method using chemical bonding may be a method using a crosslinking agent. For example, if the particle surfaces have a hydroxyl group, the carboxyl group of the specific binding substance may be reacted with a cross-linking agent to obtain an active ester, and then the hydroxyl group and the ester group may be reacted with each other, so that the specific binding substance can be immobilized on the particle surfaces. It is preferred to provide spacers between the specific binding substance and the particle surfaces so as not to inhibit the ability of the specific binding substance to recognize the target molecules.

As described above, when introducing the complex 900 into the wells 241 using a capture substance, a conjugate of the capture substance and the complex 900 is preferred to be formed under the condition that 0 or 1 unit of the complex 900 is captured by 1 molecule of the capture substance. The wells 241 are preferred to be configured so that 0 or 1 molecule of the capture substance is introduced into each well 241. Thus, digital quantification can be performed.

When the target substance 120, the first specific binding substance 140 and the second specific binding substance 170 are brought into contact with each other, a complex 900 containing these substances is formed, during which a double-stranded nucleic acid 150 is formed by hybridization of at least part of the first single-stranded nucleic acid fragment 141 with at least part of the second single-stranded nucleic acid fragment 171. The complex 900 may be formed in a sample tube or in the wells 241.

Following introduction of the mixed solution L210 into the wells 241, the openings of the wells 241 may be sealed. The method of sealing the openings of the wells 241 is not particularly limited as long as the liquid accommodated in each well 241 is not mixed with the liquid accommodated in another well 241, and for example, the openings of the wells 241 may be sealed by covering them with a sealing liquid. Alternatively, the openings of the wells 241 may be sealed by laminating a plate-like member such as a glass plate on them.

For example, as shown in Fig. 5, a sealing liquid L220 may be supplied into the channel 230 between the substrate 210 and the lid member 220 from the inlet port 222 of the lid member 220. The sealing liquid L220 supplied into the channel 230 comes into contact with the well array 240. The sealing liquid L220 expels and replaces the mixed solution L210 supplied into the channel 230 and is not accommodated in the wells 241. Thus, the sealing liquid L220 individually seals the plurality of wells 241 in which the mixed solution L210 containing the target substance 120 is accommodated, and the wells 241 become independent reaction spaces (i.e., microcompartments 242). When the channel 230 is filled with the sealing liquid L220, excess sealing liquid L220 is discharged through the outlet port 223. Fig. 6 shows a state in which all the wells 241 of the well array 240 are sealed with the sealing liquid L220, forming sealed wells (i.e., microcompartments) 242.

Alternatively, a lipid may be dissolved in the mixed solution L210 and, after supplying the sealing liquid L220 into the channel 230, the liquid containing the lipid may be additionally supplied to form a lipid bilayer membrane at the openings of the wells 241, so that the plurality of wells 241 are individually sealed with the lipid bilayer membrane, forming sealed wells 242. Examples of the lipid forming the lipid bilayer membrane include, but are not limited to, 1,2-dioleoyl-sn-glycero-3-phosphoethanolamine (DOPE), 1,2-dioleoyl-sn-glycero-3-phosphoglycerol (DOPG), and a mixture thereof.

The sealing liquid is a liquid that can form droplets (also referred to as microdroplets) by sealing the liquids introduced into the multiple wells 241 individually so that they do not mix with each other. The sealing solution is preferably an oily solution, and more preferably an oil. Examples of the oil that can be used include fluorine oils, silicone oils, hydrocarbon oils, and mixtures thereof. More specifically, FC-40 (product name) manufactured by Sigma, or the like, can be used. FC-40 (CAS Number: 86508-42-1) is a fluorinated aliphatic compound having a specific gravity at 25°C of 1.85 g/mL.

Subsequently, formation of the double-stranded nucleic acid 150 is detected. Detection of the formation of the double-stranded nucleic acid 150 is preferably performed using a signal amplification reaction. Examples of the signal amplification reaction include an Invasive Cleavage Assay (ICA).

The ICA reaction relates to the principle that signal amplification progresses through two reaction cycles which are (1) complementary binding between nucleic acids and (2) recognition and cleavage of a triple-stranded structure by enzymes.

The ICA reaction is less affected by reaction cycle inhibition due to foreign substances. Accordingly, using the ICA reaction, formation of the double-stranded nucleic acid 150 can be detected with high accuracy. If the ICA reaction is used as the signal amplification reaction, the mixed solution L210 (solution containing the MPC polymer, the target substance 120, the first specific binding substance 140 and the second specific binding substance 170) further contains a reaction reagent required for the ICA reaction.

Examples of the reaction reagent required for the ICA reaction include ICA reaction reagents, such as flap probes, flap endonucleases (FENs) and fluorescent substrates. Flap probes are nucleic acid fragments designed to hybridize with the first single-stranded nucleic acid fragment 141 or the second single-stranded nucleic acid fragment 171 to form a flap structure with the double-stranded nucleic acid 150.

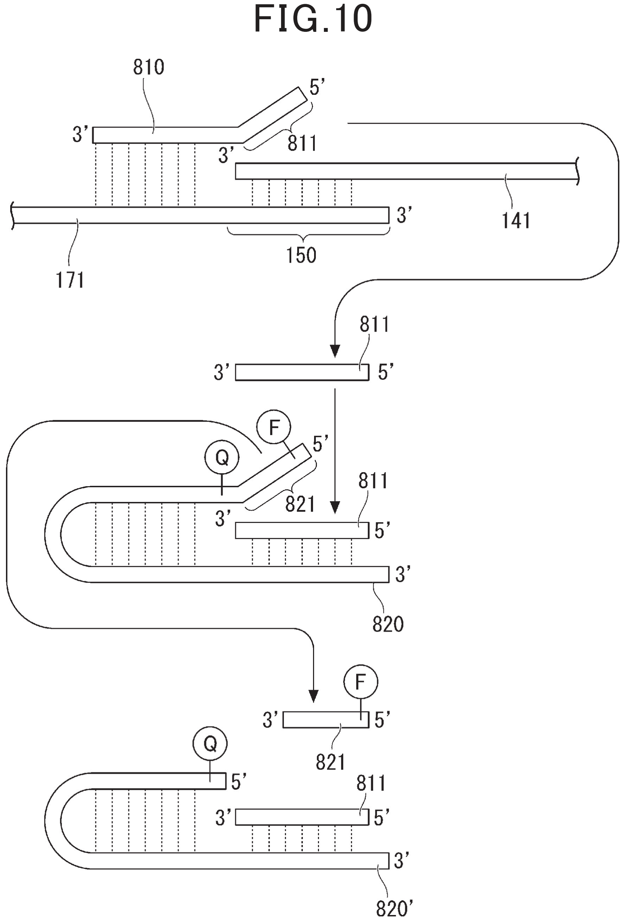

Fig. 10 is a schematic diagram illustrating an example of an ICA method. Fig. 10 shows an example of detecting a double-stranded nucleic acid 150 formed by hybridization of at least part of the first single-stranded nucleic acid fragment 141 with at least part of the second single-stranded nucleic acid fragment 171 using an ICA method.

First, a flap probe is hybridized with the first single-stranded nucleic acid fragment 141 or the second single-stranded nucleic acid fragment 171. In the example shown in Fig. 10, a flap probe 810 is hybridized with the second single-stranded nucleic acid fragment 171. Consequently, a first flap site 811 is formed.

Subsequently, an FEN is reacted with the first flap site 811, by which the first flap site 811 is cleaved to produce a nucleic acid fragment 811. Subsequently, the nucleic acid fragment 811 is hybridized with a fluorescent substrate (i.e., nucleic acid fragment 820) to form a second flap site 821.

In the example of Fig. 10, a fluorescent substance F is bound to the 5' end of the nucleic acid fragment 820, and a quenching substance Q is bound to the nucleic acid fragment 820 a few bases from the 5' end to the 3' side. Subsequently, the second flap site 821 is reacted with an FEN, by which the second flap site 821 is cleaved to produce a nucleic acid fragment 821. Consequently, the fluorescent substance F is separated from the quenching substance Q and generates a fluorescent signal. By detecting the fluorescent signal, formation of a double-stranded nucleic acid 150 can be detected.

The mixed solution L210 can be a liquid that is generally used in biochemical analyses performed using fluidic devices, and is preferred to be an aqueous solution. A surfactant or the like may be added to the mixed solution L210 to facilitate sealing of liquids in the wells.

When using the ICA reaction to detect formation of a double-stranded nucleic acid 150, if a double-stranded nucleic acid 150 is present, the fluorescent substance F is released from the quenching substance Q due to an isothermal enzymatic reaction, and a predetermined fluorescent signal is emitted in response to the excitation light.

For detecting formation of a double-stranded nucleic acid 150, a known suitable method can be selected according to the type of the signals to be detected. For example, when observing fluorescent signals, excitation light for the fluorescent substance is applied to the wells 242 to observe fluorescence emitted by the fluorescent substance. For example, as shown in Fig. 6, a predetermined reaction is allowed to occur in the sealed wells 242, and generated signals are observed. In Fig. 6, wells 242R are wells where signals have been detected, and wells 242 are wells where no signals have been detected.

Referring to Figs. 7 to 9, a method of the present embodiment will be described, taking an example of using the fluidic device 500. Here, a case where the target substance 120 is detected as in the example of Fig. 2 will be described. The method of the present embodiment is the same as that in the case where the target substance is a conjugate of a protein 110 and a protein 120, as in the example of Fig. 3, except that a second specific binding substance 130 for the protein 110 is used as the second specific binding substance.

First, as shown in Fig. 7, a mixed solution L210 is introduced into the fluidic device 500. The mixed solution L210 is a dispersion of a target substance 120, a first specific binding substance 140 and a second specific binding substance 170, and also contains a reagent for detecting formation of a double-stranded nucleic acid 150. If the sample contains the target substance 120, the mixed solution L210 contains a complex 900 containing the target substance 120, the first specific binding substance 140 and the second specific binding substance 170. In the mixed solution L210, the concentration of the complex 900 is preferred to be adjusted such that one molecule or less of the complex 900 is accommodated in each well 241.

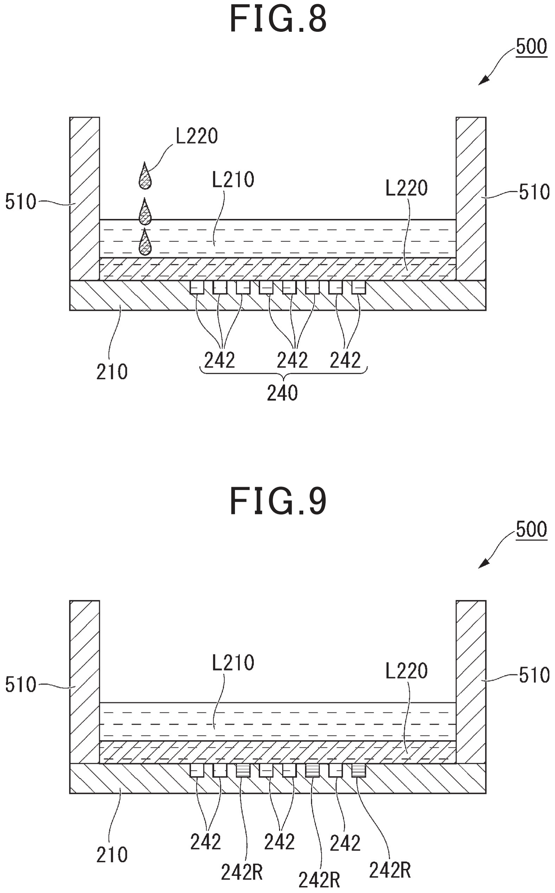

Subsequently, as shown in Fig. 8, a sealing liquid L220 is introduced into the fluidic device 500. The specific gravity of the sealing liquid L220 is greater than that of the mixed solution L210. Therefore, the sealing liquid L220 sinks below the mixed solution L210 not accommodated in the wells 241, and contacts the well array 240. Thus, the sealing liquid L220 individually seals the plurality of wells 241 in which the mixed solution L210 containing the complex 900 is accommodated, and the wells 241 become independent reaction spaces (i.e., microcompartments 242).

Subsequently, as shown in Fig. 9, a predetermined reaction is allowed to occur in the wells 242, and generated signals are observed. In Fig. 9, wells 242R are wells where signals have been detected, and wells 242 are wells where no signals have been detected.

In the embodiments shown in Figs. 2 and 3, the method of the present embodiment may include a step of synthesizing a kinase (protein 110) having VUS using a cell-free protein synthesis system and a step of detecting phosphorylation of the protein 120 in the presence of the protein 110. Alternatively, in the embodiment shown in Fig. 3, the method of the present embodiment may include a step of synthesizing a kinase (protein 110) having VUS using a cell-free protein synthesis system and a step of detecting binding between the protein 110 and the protein 120.

That is, the protein 110 may be synthesized using a cell-free protein synthesis system, and formation of the complex 900 may be performed continuously with synthesis of the protein 110. Thus, if the protein 110 is a kinase or the like containing VUS, the activity of the protein 110 can be evaluated easily and in a short period of time.

The cell-free protein synthesis system refers to a synthesis system that synthesizes proteins in vitro from template nucleic acids using ribosomes, or transcription and translation factors, etc. derived from living cells or synthesized artificially, instead of synthesizing proteins within cells.

The cell-free protein synthesis system may be a synthesis system that includes a transcription process in addition to the translation process. If the nucleic acid encoding a protein is a DNA, it is necessary to synthesize an RNA encoding the protein by transcribing the DNA. In this case, the cell-free protein synthesis system may contain factors enabling transcription. Examples of the factors enabling transcription include RNA polymerase and nucleotides, but are not limited thereto, and can include factors known to those skilled in the art.

Alternatively, an RNA may be synthesized in advance using a DNA encoding a protein as a template, and the RNA may be added to a cell-free protein synthesis system. Alternatively, an artificially chemically synthesized RNA may be used.

The nucleic acid fragment serving as a template for cell-free protein synthesis may be bio-derived, cultured cell-derived, or virus-derived. Alternatively, the nucleic acid fragment may be artificially synthesized based on genetic analysis results.

The cell-free protein synthesis system is not particularly limited, and examples thereof include synthesis systems using cell extracts obtained from wheat germ, yeast, insect cells, cultured mammalian cells, rabbit reticulocytes, Escherichia coli, etc.; and synthesis systems reconstituting factors necessary for translation. Of these synthesis systems, cell-free protein synthesis in human expression system is preferred.

The cell-free protein synthesis system may contain factors involved in translation, such as tRNA, aminoacylated tRNA synthetase, translation initiation factors, translation elongation factors, and translation termination factors.

In the above step, adenosine triphosphate (ATP) may further be brought into contact with the protein 110, the target substance 120, the first specific binding substance 140 and the second specific binding substance 170. Thus, whether the target protein is phosphorylated can be evaluated. In other words, a kinase assay can be carried out.

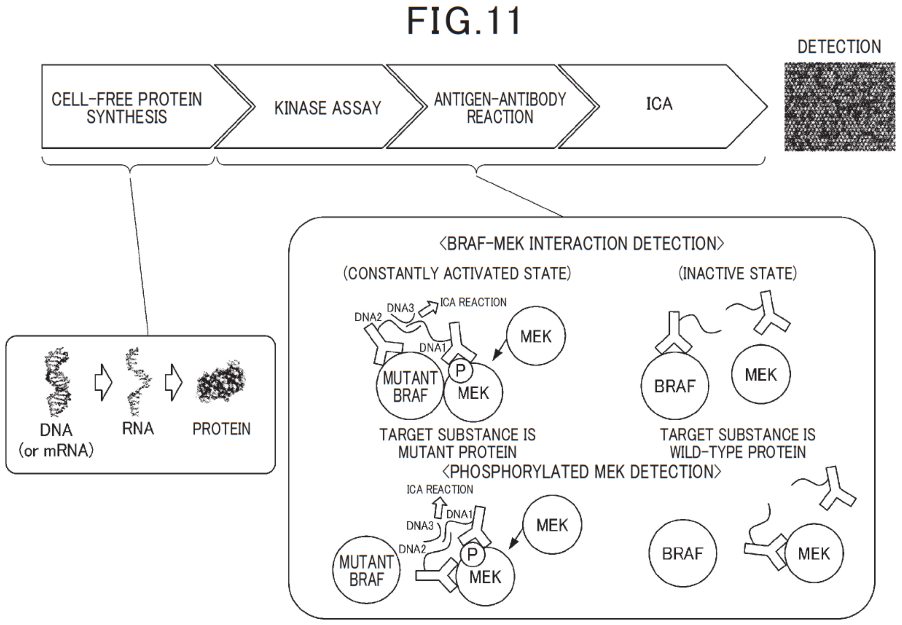

Fig. 11 is a schematic diagram illustrating an example of the method of the present embodiment. Here, as an example, when the mutant protein is BRAF, it is determined whether variants of unknown significance (VUS) in the BRAF gene cause a constantly activated state.

In Fig. 11, a cell-free protein synthesis, kinase assay, antigen-antibody reaction and ICA reaction are sequentially carried out. Thus, the activity of the protein 110 as a target protein can be evaluated more easily and in a short period of time. Also, for example, by adding an inhibitor to the reaction system, the effect of the inhibitor can also be evaluated.

It is preferred that the method of the present embodiment does not include a washing step. In particular, even in the case where the protein 110 is synthesized using a cell-free protein synthesis system in the embodiments of Figs. 2 and 3, if it is possible to evaluate whether the protein 110 binds to the protein 120 without including a washing step, the activity of the protein 110 can be evaluated even more easily and in a shorter period of time.

### [Kit]

In one embodiment, the present invention provides a kit for detecting a target substance in a sample, the kit including: (i) a well array having a plurality of wells; (ii) a first specific binding substance for the target substance, the first specific binding substance being labeled with a first single-stranded nucleic acid fragment; (iii) a second specific binding substance for the target substance, the second specific binding substance being labeled with a second single-stranded nucleic acid fragment; and (iv) a buffer containing an MPC polymer or containing casein and a nonionic surfactant.

According to the kit of the present embodiment, the above-mentioned method for detecting a target substance in a sample can be suitably carried out.

Accordingly, the kit of the present embodiment can be said to be used for the method including: a step of incubating a mixed solution containing a sample, a first specific binding substance for a target substance, the first specific binding substance being labeled with a first single-stranded nucleic acid fragment, and a second specific binding substance for the target substance, the second specific binding substance being labeled with a second single-stranded nucleic acid fragment, thereby forming a complex containing the target substance, the first specific binding substance and the second specific binding substance when the target substance is present in the sample, during which a double-stranded nucleic acid is formed by hybridization of at least part of the first single-stranded nucleic acid fragment with at least part of the second single-stranded nucleic acid fragment; and a step of detecting formation of the double-stranded nucleic acid, wherein detection of the double-stranded nucleic acid being formed indicates that the target substance is present in the sample.

The well array may be disposed inside the fluidic device described above. In the kit of the present embodiment, the target substance, the first single-stranded nucleic acid fragment, the first specific binding substance, the second single-stranded nucleic acid fragment and the second specific binding substance are the same as those which are described above.

As will be described later in the examples, when the buffer further contains an MPC polymer, it can reduce the time required for detecting the target substance.

Further, as will be described later in the examples, when the buffer further contains casein and a nonionic surfactant, it can increase the signal-to-noise ratio (S/N).

Further, as will be described later in the examples, when the buffer further contains casein, a nonionic surfactant and tris(hydroxymethyl)aminomethane, it can not only increase the signal-to-noise ratio (S/N), but also shorten the time required for detecting the target substance.

The MPC polymer, casein, nonionic surfactant and tris(hydroxymethyl)aminomethane are the same as those which are described above.

The kit of the present embodiment may further include ATP. Thus, whether the target protein is phosphorylated can be evaluated. In other words, a kinase assay can be carried out.

The kit of the present embodiment may further include a sealing liquid for sealing the openings of the wells of the well array. The sealing liquid is the same as that described above.

The kit of the present embodiment may further include a reagent for detecting a double-stranded nucleic acid formed by hybridization of at least part of the first single-stranded nucleic acid fragment with at least part of the second single-stranded nucleic acid fragment.

Examples of such a reagent include the ICA reaction reagents described above, and specific examples thereof include flap probes, flap endonucleases (FENs) and fluorescent substrates.

### Examples

### [Materials and Methods]

### (Oligonucleotide Modification to Antibodies)

Using an antibody-oligonucleotide conjugation tool (product name: oYo-Link antibody labeling reagent, manufactured by Funakoshi Co., Ltd.), different oligonucleotides (manufactured by Integrated DNA Technologies) were respectively bound to an anti-phosphorylated MEK rabbit polyclonal antibody (anti-pMEK1/2 (S217/221) rabbit antibody, manufactured by Cell Signaling Technology, Inc.) and an anti-MEK rabbit monoclonal antibody (anti-MEK1/2 rabbit antibody, manufactured by Cell Signaling Technology, Inc.). Specifically, oligonucleotide DNA1 (5'-TTTGTCACTGTTCCTCCTTTTGTTTTCCTTTCTGTGAGCAATTTCACCCAA-3', Seq. No. 1) was bound to the anti-phosphorylated MEK rabbit polyclonal antibody to obtain a DNA1-modified anti-phosphorylated MEK rabbit polyclonal antibody. Further, oligonucleotide DNA2 (5'-GCATGGTTCCAATTTGGGTGAT-3', Seq. No. 2) was bound to the anti-MEK rabbit polyclonal antibody to obtain a DNA2-modified anti-MEK rabbit polyclonal antibody.

### (Preparation of Antigen-Antibody Reaction Solution)

The reagents listed in Table 1 below were mixed to prepare an antigen-antibody reaction solution. In Table 1, "BSA" represents bovine serum albumin, and "TBS" represents Tris-buffered saline.

**[Table 1]**

| Antigen-antibody reaction solution | |
|---|---|
| Reagent | Final concentration |
| DNA1-modified anti-phosphorylated MEK rabbit polyclonal antibody | 8.56 nM |
| DNA2-modified anti-MEK rabbit polyclonal antibody | 8.56 nM |
| Phosphorylated MEK or non-phosphorylated MEK | 28,210 pM |
| Blocking buffer (1% BSA-TBS) | - |

### (Study on MPC Polymer)

Seven types of MPC polymers were respectively added to the above-mentioned antigen-antibody reaction solution to a final concentration of 0.5% (w/v). The MPC polymers used are shown in Table 2 below. In Table 2, the surface tension is a value measured at 25°C using a 0.1 wt% aqueous solution, and the kinematic viscosity is a value measured at 25°C using 1 wt% aqueous solution.

**[Table 2]**

| Product name | Surface tension (× 10⁻³ N/m) | Kinematic viscosity (cSt) |
|---|---|---|
| Lipidure (registered trademark)-BL103, NOF | approx. 72 | approx. 13 |
| Lipidure (registered trademark)-BL203, NOF | approx. 53 | approx. 21 |
| Lipidure (registered trademark)-BL206, NOF | approx. 39 | approx. 1 |
| Lipidure (registered trademark)-BL405, NOF | approx. 73 | approx. 5 |
| Lipidure (registered trademark)-BL802, NOF | approx. 45 | approx. 2 |
| Lipidure (registered trademark)-BL1002, NOF | approx. 63 | approx. 2 |
| Lipidure (registered trademark)-BL1003, NOF | approx. 56 | approx. 1 |

### (Study on Blocking Buffer)

Instead of the blocking buffer (1% BSA-TBS), commercially available blocking buffers listed in Table 3 below were used.

**[Table 3]**

| Composition | Product name |
|---|---|
| Blocking buffer containing casein, nonionic surfactant and tris(hydroxymethyl)aminomethane (pH 7 to 8) | Can Get Signal buffer solution A, Toyobo |
| Blocking buffer containing casein, nonionic surfactant and sodium dihydrogenphosphate (pH 7 to 8) | Can Get Signal buffer solution B, Toyobo |

### (Preparation of ICA Reaction Solutions)

ICA reaction solutions for performing an ICA reaction were prepared using the oligonucleotides modified to the above antibodies. Table 4 below shows the composition of the ICA reaction solution.

**[Table 4]**

| ICA reaction solution | |
|---|---|
| Reagent | Final concentration |
| Allele probe (5'-CGCGCCGAGGAATTGCTCACAGAAAGGA-3') (Fasmac Co., Ltd., Seq. No. 3) | 2 µM |
| FRET Cassette (fluorescent substrate, Alexa 488-BHQ: 5'-X-TTCT-Y-AGCCGGTTTTCCGGCTGAGACCTCGGCGCG-3', X: Alexa 488 + Amino C6, Y: Black hole quencher (BHQ) 1-dT) (Japan Bio Services, Seq. No. 4) | 4 µM |
| Flap endonuclease (FEN)-1 | 0.216 µM |
| Tris-HCl (pH 8.5) | 50 mM |
| MgCl₂ | 20 mM |
| Tween 20 | 0.05% |

### (Antigen-Antibody Reaction and ICA Reaction)

The above-mentioned antigen-antibody reaction solution and the above-mentioned ICA reaction solution were mixed together, and incubated at 30°C for 60 minutes. Subsequently, the mixture was incubated at 66°C for 60 minutes to perform an ICA reaction.

### [Experimental Example 1]

### (Study on MPC Polymer)

10 µL of the above-mentioned antigen-antibody reaction solution (containing MPC polymer) and 10 µL of the above-mentioned ICA reaction solution were mixed together, and incubated at 30°C for 60 minutes. Subsequently, the mixture was incubated at 66°C for 60 minutes using Rotor-Gene Q (Qiagen) to detect Alexa 488 fluorescence. This reaction may hereinafter be referred to as an immuno-ICA reaction.

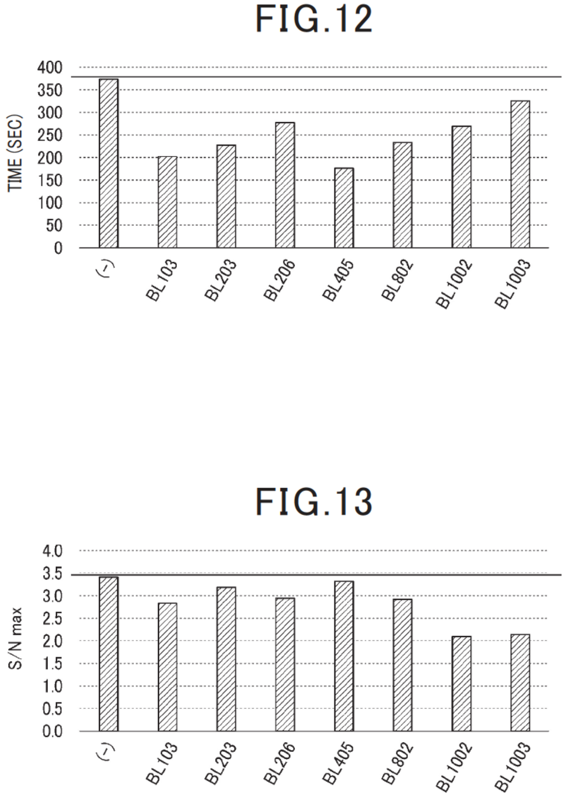

Figs. 12 and 13 are graphs showing the results of the immuno-ICA reaction. The fluorescence intensity detected by the immuno-ICA reaction using phosphorylated MEK was defined as a signal (S) and the fluorescence intensity detected by the immuno-ICA reaction using non-phosphorylated MEK was defined as a noise (N), and the S/N was calculated.

Fig. 12 is a graph showing the results of examining the effect of the addition of the MPC polymer on the reaction rate. In Fig. 12, the vertical axis indicates the time required for the S/N to reach the maximum value, and the horizontal axis indicates the type of the added MPC polymer. "BL103", "BL203", "BL206", "BL405", "BL802", "BL1002" and "BL1003" respectively refer to Lipidure (registered trademark)-BL103, Lipidure (registered trademark)-BL203, Lipidure (registered trademark)-BL206, Lipidure (registered trademark)-BL405, Lipidure (registered trademark)-BL802, Lipidure (registered trademark)-BL1002 and Lipidure (registered trademark)-BL1003 (all manufactured by NOF Corporation). Further, (-) indicates the result of a sample to which no MPC polymer was added for comparison. As a result, it was found that the time required for the S/N to reach the maximum value can be shortened by adding the MPC polymer to the reaction solution.

Fig. 13 is a graph showing the results of examining the effect of the addition of the MPC polymer on S/N. In Fig. 13, the vertical axis indicates the maximum S/N value. Further, the horizontal axis, as in Fig. 12, indicates the type of the added MPC polymer. As a result, even when the MPC polymer was added to the reaction solution, no improvement in S/N was observed.

### [Experimental Example 2]

### (Study on Blocking Buffer)

10 µL of the antigen-antibody reaction solution in which the type of blocking buffer was changed to the above-mentioned blocking buffer, and 10 µL of the above-mentioned ICA reaction solution were mixed together, and incubated at 30°C for 60 minutes. Subsequently, the mixture was incubated at 66°C for 60 minutes using Rotor-Gene Q (Qiagen) to detect Alexa 488 fluorescence.

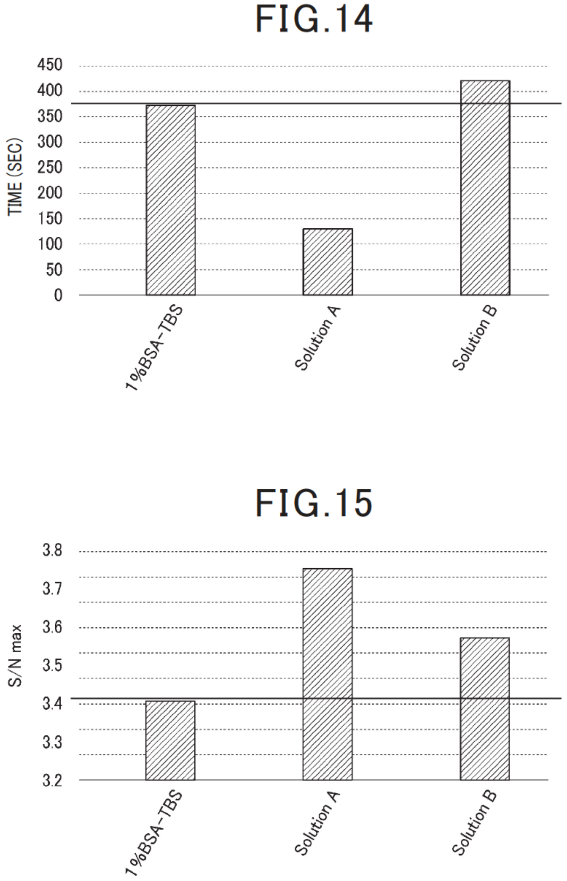

Figs. 14 and 15 are graphs showing the results of the immuno-ICA reaction. The fluorescence intensity detected by the immuno-ICA reaction using phosphorylated MEK was defined as a signal (S) and the fluorescence intensity detected by the immuno-ICA reaction using non-phosphorylated MEK was defined as a noise (N), and the S/N was calculated.

Fig. 14 is a graph showing the results of examining the effect of the type of blocking buffer on the reaction rate. In Fig. 14, the vertical axis indicates the time required for the S/N to reach the maximum value, and the horizontal axis indicates the type of the used blocking buffer. "Solution A" refers to Can Get Signal buffer solution A (Toyobo), and "Solution B" refers to Can Get Signal buffer solution B (Toyobo). As a result, it was found that the time required for the S/N to reach the maximum value can be significantly shortened by using a blocking buffer (Can Get Signal buffer solution A) containing casein, a nonionic surfactant and tris(hydroxymethyl)aminomethane instead of 1% BSA-TBS as the blocking buffer.

Fig. 15 is a graph showing the results of examining the effect of the type of blocking buffer on S/N. In Fig. 15, the vertical axis indicates the maximum S/N value, and the horizontal axis, as in Fig. 14, indicates the type of the used blocking buffer. As a result, it was found that the maximum S/N value can be increased by using a blocking buffer (Can Get Signal buffer solution A or Can Get Signal buffer solution B, both manufacture by Toyobo) containing casein and a nonionic surfactant instead of 1% BSA-TBS as the blocking buffer.

### [Experimental Example 3]

### (Effect of MPC Polymer on ICA Reaction)

Lipidure (registered trademark)-BL1002 or Lipidure (registered trademark)-BL1003 (both manufactured by NOF Corporation) was added as an MPC polymer to the ICA reaction solution shown in Table 5 to a final concentration of 0.5% (w/v), 0.05 mass% (w/v) or 0.01 mass% (w/v), and the mixture was incubated at 66°C for 60 minutes to perform an ICA reaction.

**[Table 5]**

| Reagent | Final concentration |
|---|---|
| Allele probe 2 (5'-CGCGCCGAGGCGCAGCTCATGCCC-3') (manufactured by Fasmac Co., Ltd., Seq. No. 5) | 1 µM |
| 1-1 alexa 488 | 4 µM |
| NaCl | 20 mM |
| MgCl₂ | 25.0 mM |
| Tween 20 | 0.05% |
| Tris-HCl (pH 8.5) | 50 mM |
| TKO-Fen1 | 5.16 µM |
| Target DNA (5'- GCCAGGAACGTACTGGTGAAAACACCGCAGCATGTCAAGATCACAGA TTTTGGGCTGGCCAAACTGCTGGGTGCGGAAGAGAAAGAATACCATG CAGAAGG-3') (manufactured by Fasmac Co., Ltd., Seq. No. 6) | 1.5 pM |
| Invader DNA (5'-TCTGCCTCACCTCCACCGTGCARCTCATCAA-3') (manufactured by Fasmac Co., Ltd., Seq. No. 7) | 10 nM |

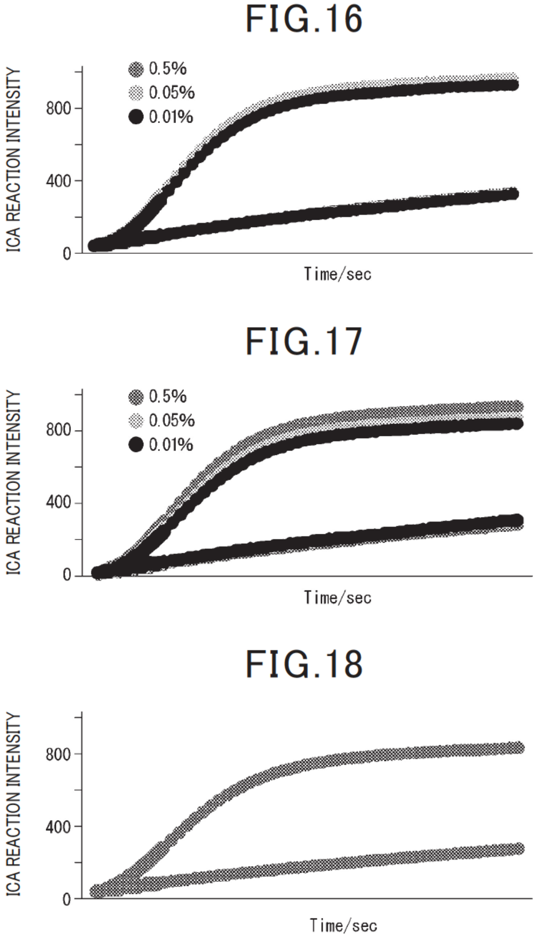

Figs. 16 and 17 are graphs showing the results of examining the effect of the addition of Lipidure (registered trademark)-BL1002 and Lipidure (registered trademark)-BL1003, respectively, on ICA reactivity. In Figs. 16 and 17, the vertical axis indicates the ICA reaction intensity. The horizontal axis indicates the reaction time. Further, Fig. 18 shows the results of a sample to which no MPC polymer was added for comparison. As a result, when the MPC polymer was added to the reaction solution, an increase in the reaction intensity was observed.

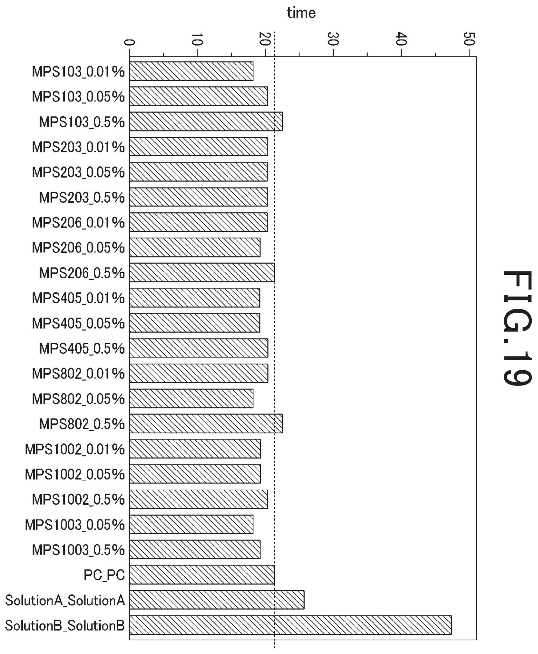

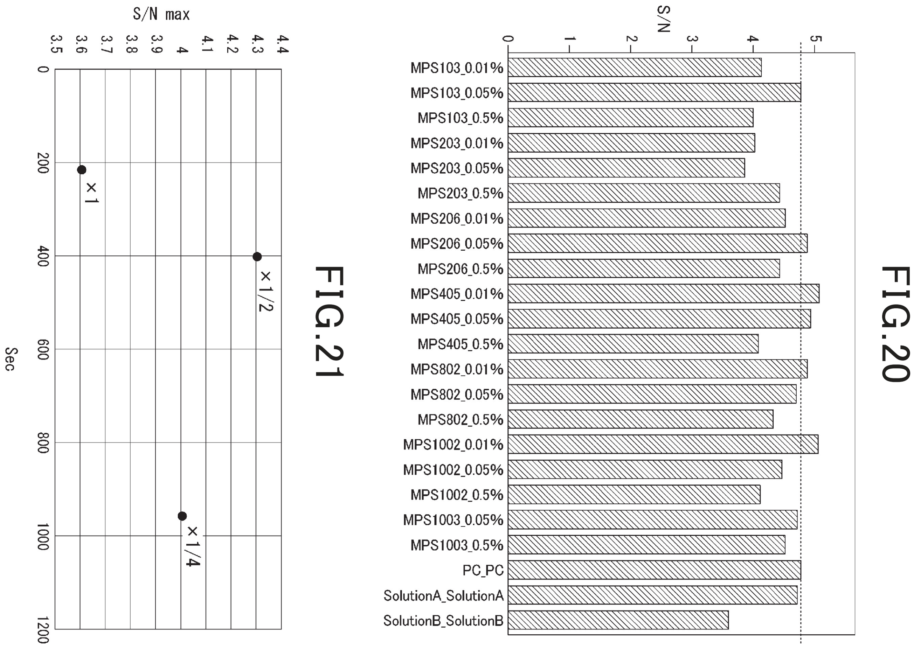

Further, under the same conditions, an ICA reaction was performed by adding the MPC polymer shown in Table 2 or the blocking buffer shown in Table 3. The fluorescence intensity when the target DNA was added was defined as a signal (S) and the fluorescence intensity when the target DNA was not added was defined as a noise (N), and the S/N was calculated. Fig. 19 shows the time (unit: seconds) required for the S/N to reach the maximum value when the MPC polymer shown in Table 2 or the blocking buffer shown in Table 3 was added. Fig. 20 shows the maximum S/N value when the MPC polymer shown in Table 2 or the blocking buffer shown in Table 3 was added. In Figs. 19 and 20, PC indicates the result of a sample to which no MPC polymer or blocking buffer was added for comparison.

When the MPC polymer or the blocking buffer shown in Table 3 was added, both the maximum S/N value and the time required for the S/N to reach the maximum value were either comparable or inferior to those of PC. Therefore, it was suggested that the addition of the MPC polymer or the blocking buffer shown in Table 3 did not improve the ICA reaction, but improved the reactivity of the immuno-ICA.

### [Experimental Example 4]

### (Effect of Blocking Buffer on ICA Reaction)

The effect of the buffer concentration on the reactivity when adding the blocking buffer containing casein, a nonionic surfactant and tris(hydroxymethyl)aminomethane, shown in Table 3, was examined. Specifically, a complex was formed under the antigen-antibody reaction conditions of the ELISA reaction, and an ICA reaction was performed.

### (Oligonucleotide Modification to Antibodies)

Using an antibody-oligonucleotide conjugation tool (product name: oYo-Link antibody labeling reagent, manufactured by Funakoshi Co., Ltd.), different oligonucleotides (manufactured by Integrated DNA Technologies) were respectively bound to an anti-phosphorylated MEK rabbit polyclonal antibody (anti-pMEK1/2 (S217/221) rabbit antibody, manufactured by Cell Signaling Technology, Inc.) and an anti-MEK rabbit monoclonal antibody (anti-MEK1/2 rabbit antibody, manufactured by Cell Signaling Technology, Inc.). Specifically, oligonucleotide DNA11 (5'-TTTGTCACTGTTCCTCCTTTTGTTTTCCTTTCTGTGAGCAATTTCACCCAA-3', Seq. No. 9) was bound to the anti-phosphorylated MEK rabbit polyclonal antibody to obtain a DNA11-modified anti-phosphorylated MEK rabbit polyclonal antibody. Further, oligonucleotide DNA12 (5'-GCATGGTTCCAATTTGGGTGAT-3', Seq. No. 10) was bound to the anti-MEK rabbit polyclonal antibody to obtain a DNA12-modified anti-MEK rabbit polyclonal antibody.

### (Kinase Assay and Antigen-Antibody Reaction)

The reagents listed in Table 6 below were mixed to prepare 10 µL of an antigen-antibody reaction solution.

**[Table 6]**

| Reagent | Final concentration |
|---|---|
| DNA11-modified anti-phosphorylated MEK rabbit polyclonal antibody | 8.56 nM |
| DNA12-modified anti-MEK rabbit polyclonal antibody | 8.56 nM |
| BRAF (1000 ng/mL) Mt or WT (manufactured by Oligene) | 2821 pM |
| Phosphorylated MEK or non-phosphorylated MEK | 28210 pM |
| ATP (500 µM) | 50 µM |
| Blocking buffer | - |

### (Preparation of ICA Reaction Solutions)

ICA reaction solutions for performing an ICA reaction were prepared using the oligonucleotides modified to the above antibodies. Table 7 below shows the composition of the ICA reaction solution.

**[Table 7]**

| Reagent | Final concentration |
|---|---|
| Allele probe 3 (5'-CGCGCCGAGGAATTGCTCACAGAAAGGA-3') (manufactured by Fasmac Co., Ltd., Seq. No. 8) | 1 µM |
| 1-1 alexa 488 | 4 µM |
| MgCl₂ | 20.0 mM |
| Tween 20 | 0.05% |
| Tris-HCl (pH 8.5) | 50 mM |
| TKO-Fen1 | 0.216 µM |
| DNA11 (5'- TTTGTCACTGTTCCTCCTTTTGTTTTCCTTTCTGTGAGCAATTTC ACCCAA3', Seq. No. 9) (manufactured by Integrated DNA Technologies Inc.) | 8.56 nM |
| DNA12 (5'-GCATGGTTCCAATTTGGGTGAT-3' Seq. No. 10) (manufactured by Integrated DNA Technologies Inc.) | 8.56 nM |

### (Antigen-Antibody Reaction and ICA Reaction)

The above-mentioned kinase assay and antigen-antibody reaction solution and the above-mentioned ICA reaction solution were mixed together, and incubated at 30°C for 60 minutes. Subsequently, the mixture was incubated at 66°C for 60 minutes to perform an ICA reaction.

The concentration of the blocking buffer containing casein, a nonionic surfactant and tris(hydroxymethyl)aminomethane was set to 1, and reaction solutions adjusted to concentrations of 1/2 and 1/4 were prepared, and measurement was performed using a real-time PCR device (Rotor-Gene Q, manufactured by Qiagen). The fluorescence intensity detected by the immuno-ICA reaction using phosphorylated MEK was defined as a signal (S) and the fluorescence intensity detected by the immuno-ICA reaction using non-phosphorylated MEK was defined as a noise (N), and the S/N was calculated.

Fig. 21 is a graph showing the results of examining the effect of the blocking buffer concentration on S/N. In Fig. 21, the vertical axis indicates the maximum S/N value, and the horizontal axis indicates the time required for the S/N to reach the maximum value.

When the concentration of the blocking buffer containing casein, a nonionic surfactant and tris(hydroxymethyl)aminomethane was 1/2, the maximum S/N value (4.3) was reached. However, the time required to reach the maximum S/N value was doubled compared to the conditions of ×1.

### [Industrial Applicability]

According to the present invention, a method for detecting a target substance in a sample and a kit therefor can be provided.

### [Reference Signs List]

100, 900 ... Complex
110, 120 ... Target substance (protein)
160 ... Phosphate group
130, 140, 170 ... Specific binding substance
131, 141, 171 ... Single-stranded nucleic acid fragment
150 ... Double-stranded nucleic acid (double-stranded nucleic acid region)
200, 500 ... Fluidic device
210 ... Substrate
220 ... Lid member
221 ... Projection
222 ... Inlet port
223 ... Outlet port
230 ... Channel
241, 242, 242R ... Well (microcompartment)
240 ... Well array
510 ... Wall member
810 ... Flap probe
811, 821 ... Flap site (nucleic acid fragment)
820 ... Nucleic acid fragment

## Claims

1. A method for detecting a target substance in a sample, the method comprising:
a step of incubating a mixed solution containing the sample, a first specific binding substance for the target substance, the first specific binding substance being labeled with a first single-stranded nucleic acid fragment, and a second specific binding substance for the target substance, the second specific binding substance being labeled with a second single-stranded nucleic acid fragment, thereby forming a complex containing the target substance, the first specific binding substance and the second specific binding substance when the target substance is present in the sample, during which a double-stranded nucleic acid is formed by hybridization of at least part of the first single-stranded nucleic acid fragment with at least part of the second single-stranded nucleic acid fragment; and
a step of detecting formation of the double-stranded nucleic acid, wherein
detection of the double-stranded nucleic acid being formed indicates that the target substance is present in the sample, and
the mixed solution further contains 2-methacryloyloxyethyl phosphorylcholine copolymer, or the mixed solution further contains casein and a nonionic surfactant.

2. The method according to claim 1, wherein
the 2-methacryloyloxyethyl phosphorylcholine copolymer is a copolymer of 2-methacryloyloxyethyl phosphorylcholine and a comonomer having at least one group selected from the group consisting of a hydrophobic group, a hydrophilic group, an anionic group and a cationic group.

3. The method according to claim 1 or 2, wherein
the 2-methacryloyloxyethyl phosphorylcholine copolymer in the mixed solution has a concentration of 0.01 to 0.5% (w/v).

4. The method according to claim 1 or 2, wherein
the incubation of the mixed solution is performed in a well, and the well has a volume of 10 fL to 100 pL.

5. The method according to claim 1 or 2, wherein
the first single-stranded nucleic acid fragment and the second single-stranded nucleic acid fragment each have a length of 10 to 200 bases.

6. The method according to claim 1 or 2, wherein
the step of detecting formation of the double-stranded nucleic acid is performed through an invasive cleavage assay.

7. A kit for detecting a target substance in a sample, the kit comprising:
a well array having a plurality of wells;
a first specific binding substance for the target substance, the first specific binding substance being labeled with a first single-stranded nucleic acid fragment;
a second specific binding substance for the target substance, the second specific binding substance being labeled with a second single-stranded nucleic acid fragment; and
a buffer containing 2-methacryloyloxyethyl phosphorylcholine copolymer or containing casein and a nonionic surfactant.

8. The kit according to claim 7, further comprising a sealing liquid for sealing openings of the wells.

9. The kit according to claim 7 or 8, further comprising a reagent for detecting a double-stranded nucleic acid formed by hybridization of at least part of the first single-stranded nucleic acid fragment with at least part of the second single-stranded nucleic acid fragment.
